# EUROPÄISCHE PATENTSCHRIFT

(11) **EP 4 231 961 B1**
(45) Veröffentlichungstag und Bekanntmachung des Hinweises auf die Patenterteilung: **18.12.2024**
(21) Anmeldenummer: 21802600.3
(22) Anmeldetag: 21.10.2021
(51) Int. Cl.: A61F 2/07, A61F 2/856

(54) **MEDIZINISCHES IMPLANTAT ZUR BEHANDLUNG VON ANEURYSMEN**
MEDICAL IMPLANT FOR TREATING ANEURYSMS
IMPLANT MÉDICAL POUR LE TRAITEMENT D'ANÉVRISMES

(30) Priorität: 26.10.2020 DE 102020128124
(43) Veröffentlichungstag der Anmeldung: 30.08.2023
(73) Patentinhaber: Acandis GmbH, 75177 Pforzheim (DE)
(72) Erfinder: BÜCHERT, Michael, 75177 Pforzheim (DE); SCHÜSSLER, Andreas, 76327 Pfinztal (DE)
(74) Vertreter: Meissner Bolte Partnerschaft mbB
(86) Internationale Anmeldenummer: PCT/EP2021/079204
(87) Internationale Veröffentlichungsnummer: WO 2022/090049

(56) Entgegenhaltungen:
- EP-B1- 2 678 466
- WO-A1-2007/094738
- WO-A1-2011/076408
- WO-A1-2019/175341
- WO-A2-2006/034114
- WO-A2-2006/123340
- DE-A1- 102010 026 830
- DE-A1- 102018 110 591
- DE-A1- 102018 131 269
- US-A1- 2007 031 607
- US-A1- 2014 358 221

## Beschreibung

Die Erfindung betrifft ein medizinisches Implantat zur Behandlung von Aneurysmen gemäß dem Oberbegriff des Patentanspruchs 1. Ein derartiges Implantat ist beispielsweise aus EP 2 678 466 B1, US 2014/358221 A1 oder DE 10 2018 131269 A1 bekannt.

EP 2 678 466 B1 befasst sich mit einem Stent für neurovaskuläre Anwendungen, der mit einem Faservlies bedeckt ist. Das Faservlies wird durch Elektrospinnen hergestellt und umfasst mehrere Schichten, wobei eine innere Schicht flüssigkeitsundurchlässig und eine äußere Schicht schwammförmig ausgebildet ist. Die innere Schicht dient dazu, ein Aneurysma von einem Blutstrom in einem Blutgefäß abzukapseln. Die äußere, schwammförmige Schicht soll das Einlagern von Endothelzellen und/oder Medikamenten ermöglichen. Der Nachteil bei dem bekannten Implantat besteht darin, dass das Implantat nur für Aneurysmen eingesetzt werden kann, die entfernt von einem abzweigenden Blutgefäß, von Arterien oder von Arteriolen angeordnet sind. Bei Aneurysmen, die nahe einer Gefäßabzweigung platziert sind, besteht beim Einsatz des bekannten Implantats die Gefahr, dass auch das abzweigende Blutgefäß vom Blutstrom abgetrennt wird.

In der Folge kann es zu einem erheblichen Versorgungsmangel in Gewebebereichen kommen, die von dem abzweigenden Blutgefäß mit Sauerstoff und Nährstoffen versorgt werden sollen.

Der Erfindung liegt vor diesem Hintergrund die Aufgabe zu Grunde, ein medizinisches Implantat zur Behandlung von Aneurysmen anzugeben, das einerseits eine effiziente Abdeckung eines Aneurysmas ermöglicht und andererseits sicherstellt, dass die Blutzufuhr in abzweigende Blutgefäße weiterhin gewährleistet ist.

Diese Aufgabe wird durch den Gegenstand des Patentanspruchs 1 gelöst.

So beruht die Erfindung auf dem Gedanken, ein medizinisches Implantat zur Behandlung von Aneurysmen mit einer Trägerstruktur anzugeben, die eine komprimierbare und expandierbare Gitterstruktur aus Gitterelementen aufweist, welche Gitteröffnungen begrenzen. Die Gitterstruktur ist zumindest abschnittsweise mit einer aus Fasern gebildeten oder bestehenden Membran abgedeckt. Die Membran umfasst mindestens eine luminale Funktionsschicht und mindestens eine abluminale Stützschicht, die jeweils Poren aufweisen. Die Porosität der Funktionsschicht ist dabei kleiner als die Porosität der Stützschicht. Erfindungsgemäß ist vorgesehen, dass die Membran so konfiguriert ist, dass sich zumindest die Poren der luminalen Funktionsschicht der Membran infolge eines Druckgradienten, der sich zwischen einem Flüssigkeitsdruck in einem inneren Durchgangskanal der Trägerstruktur und einem Flüssigkeitsdruck außerhalb der abluminalen Stützschicht einstellt, zur Erhöhung des Flüssigkeitsdurchflusses durch die Membran öffnen.

Die Erfindung geht somit auf die Idee zurück, die Membran intelligent wirken zu lassen, so dass diese dann höher flüssigkeitsdurchlässig wird, wenn ein Druckgradient zwischen einem Flüssigkeitsdruck im inneren Durchgangskanal der Trägerstruktur und einem Flüssigkeitsdruck außerhalb der Membran besteht. Im implantierten Zustand öffnen sich die Poren der Membran, insbesondere in der Funktionsschicht, wenn die Membran, die mit dem Implantat in einem Hauptblutgefäß eingesetzt ist, ein abzweigendes Blutgefäß überspannt und so ein Druckgradient zwischen dem Hauptblutgefäß, insbesondere einer Arterie, und einer davon abzweigenden Arterie oder Arteriole vorliegt. Das durch das Hauptgefäß in das abzweigende Blutgefäß, insbesondere die abzweigende Arterie oder die abzweigende Arteriole, fließende Blut hat einen Flüssigkeitsdruck, der das Blut in das abzweigende Blutgefäß beziehungsweise abzweigende Arteriolen drängt. Die innere Membran ist also so ausgelegt, dass dieser Flüssigkeitsdruck ausreicht, um die Poren zumindest der Funktionsschicht der Membran lokal so weit zu öffnen, dass diese an Stellen, an welchen Blutgefäße, insbesondere Arterien oder Arteriolen, abzweigen, blutdurchlässig wird. Insbesondere ist die Membran so angepasst, dass sie so blutdurchlässig wird, dass durch die abzweigenden Blutgefäße oder Arteriolen angebundene Gewebeareale ausreichend mit Sauerstoff und Nährstoffen versorgt werden.

In Bereichen, in welchen das Implantat ein Aneurysma abdeckt, ist ein solcher Druckgradient jedoch nicht vorhanden, so dass die Poren der Membran soweit verschlossen bleiben, wodurch das Aneurysma vom Blutfluss innerhalb des Blutgefäßes effizient abgeschirmt wird. Jedenfalls ist vorgesehen, dass die Abschirmung effizienter erfolgt, als dies bei bisher bekannten bzw. konventionellen Flow Diverter Implantaten der Fall ist.

Die Abschirmung muss dabei nicht im Sinne einer vollständigen Flüssigkeitsbarriere erfolgen. Vielmehr kann ein stark reduzierter Flüssigkeitsaustausch zwischen dem Blut innerhalb des Aneurysmas und dem Blut innerhalb des Hauptgefäßes bestehen bleiben. Die Abschirmung des Aneurysmas reicht jedoch aus, um die Blutströmung innerhalb des Aneurysmas so zu reduzieren, dass das Blut innerhalb des Aneurysmas gerinnt und sich so ein Thrombus innerhalb des Aneurysmas bildet. Insofern wird das Aneurysma natürlich verödet, wobei die Membran des Implantats dafür Sorge trägt, dass der Thrombus das Aneurysma nicht verlässt. Die Bildung eines Thrombus reduziert den Druck auf die Aneurysmenwand, so dass die Gefahr einer Aneurysmenruptur und ein damit einhergehender Blutverlust bzw. blutiger Schlaganfall reduziert werden.

Der Vorteil des erfindungsgemäßen Implantats liegt auf der Hand. Bei der Behandlung von Aneurysmen, insbesondere zerebralen Aneurysmen, mittels des erfindungsgemäßen Implantats ist es für einen Operateur nicht zwingend erforderlich, die Position des Implantats exakt zu bestimmen. Insbesondere muss der Operateur nicht darauf achten, ein Implantat in einer bestimmten Länge einzusetzen, um das Überdecken von abzweigenden Blutgefäßen zu verhindern. Dies gilt insbesondere auch für kleinere, abzweigende Blutgefäße, die sog. "perforating arteries", welche typischerweise einen Durchmesser zwischen 300 µm und 850 µm aufweisen, und abzweigende Arteriolen, die meist einen Durchmesser zwischen 50 µm und 300 µm haben. Vielmehr ist es möglich, das medizinische Implantat in einer Standardlänge für jede Art von Aneurysmen einzusetzen, da das Implantat einen Blutfluss in abzweigende Blutgefäße, insbesondere Arterien bzw. Arteriolen, auch dann ermöglicht, wenn das Implantat das abzweigende Blutgefäß, insbesondere Arterien und abzweigende Arteriolen, überdeckt. Dies erleichtert und beschleunigt die Behandlung von Aneurysmen erheblich.

Vorzugsweise umfasst die Membran ein elektrogesponnenes Gewebe, das die Fasern bildet. Das Gewebe ist vorzugsweise mehrschichtig und ist aus Fasern mit unterschiedlichen Eigenschaften gebildet. Insbesondere kann das Gewebe die Stützschicht und die Funktionsschicht umfassen, die jeweils aus Fasern gebildet sind, wobei die Fasern unterschiedliche Eigenschaften, insbesondere eine unterschiedliche Elastizität und/oder Faserdicke, aufweisen. Die Membran weist vorzugweise auf der luminalen Seite, also der dem Hauptgefäßlumen zugewandten Seite, eine relativ niedrige Porosität auf, welche sich unter einem Druckgradienten vergrößert (und somit die eigentliche Funktionalität bildet) und umfasst auf der abluminalen Seite, also der der Gefäßwand zugewandten Seite, eine relativ höhere Porosität, die stützend wirkt. Insofern unterscheidet die vorliegende Anmeldung zwischen der luminalen Funktionsschicht, die die Funktionalität der intelligent wirkenden Membran bestimmt, und der abluminalen Stützschicht, die die Funktionsschicht stützt. Die Stützschicht verändert ihre Porosität unter dem Einfluss eines durch ein abzweigendes Blutgefäß oder eine abzweigende Arteriole entstehenden Druckgradienten im Wesentlichen nicht oder zumindest kaum. Die Stützschicht kann außerdem so ausgelegt sein, dass sie ein Ausweichen der Fasern der Funktionsschicht in radiale Richtung verhindert.

Die Funktionsschicht und die Stützschicht können sich insbesondere durch die Art der Faseranordnung unterscheiden. Vorzugsweise umfasst oder besteht die Stützschicht aus Fasern, die überwiegend miteinander vernetzt sind und so eine stabile Schicht bilden. Die Bewegungsfreiheit der Fasern der Stützschicht ist durch die Vernetzung begrenzt. Die Funktionsschicht hingegen kann aus Fasern gebildet sein oder Fasern umfassen, die überwiegend lose aufeinanderliegen. Das ermöglicht eine höhere Bewegungsfreiheit der Fasern, so dass sich durch Faserverschiebung größere Poren bilden lassen. Vorzugsweise weisen die Fasern der Stützschicht einen relativ größeren Durchmesser und eine relativ höhere Festigkeit bzw. Shore-Härte auf, wogegen die Fasern der Funktionsschicht einen relativ kleineren Durchmesser und eine relativ geringere Festigkeit bzw. Shore-Härte haben können.

Grundsätzlich kann es dabei vorgesehen sein, dass die Funktionsschicht und die Stützschicht deutlich voneinander abgegrenzte Schichten bilden. Es ist jedoch möglich, dass die Funktionsschicht und die Stützschicht nicht klar voneinander abgrenzbar sind, sondern diese Bereiche unterschiedlicher Porosität fließend ineinander übergehen. Die Membran bildet vorzugsweise einen Kompromiss zwischen Funktionalität (Öffnen bzw. nicht Öffnen der Poren durch Bewegung oder Verformung der Fasern) und Stützwirkung. Dabei können die Bereiche unterschiedlicher Porosität, insbesondere die Funktionsschicht und die Stützschicht so ineinander verschwimmen, dass die Funktionsschicht und die Stützschicht in Richtung des Druckgradienten nicht voneinander unterscheidbar sind. Vielmehr kann die Membran insgesamt in Richtung des Druckgradienten eine nahezu gleiche Porosität aufweisen. Vorgenanntes gilt für den unbelasteten Zustand der Membran, d.h. ohne Einwirkung des Druckgradienten. Unter Einwirkung des Druckgradienten bewegen oder verformen sich hingegen einige der Fasern der Funktionsschicht der Membran, so dass größere Poren entstehen und die Membran somit für Blut so weit durchlässig wird, dass abzweigende Blutgefäße ausreichend Sauerstoff und Nährstoffe in nachgelagerte Gewebeareale fördern können. Die Verformung kann eine elastische oder eine plastische Verformung sein. Jedenfalls ist vorgesehen, dass die Entstehung der größeren Poren ohne Faserrisse erfolgt. Vielmehr kann die Vergrößerung der Poren zerstörungsfrei erfolgen und ebenso zerstörungsfrei rückgängig gemacht werden, beispielsweise wenn sich der Druckgradient zwischen dem Flüssigkeitsdruck im inneren Durchgangskanal der Trägerstruktur und dem Flüssigkeitsdruck außerhalb der Stützschicht reduziert.

Bevorzugt ist es, wenn die Funktionsschicht und die Stützschicht stoffschlüssig miteinander verbunden sind. Insbesondere können sich einzelne Fasern der Funktionsschicht und der Stützschicht überkreuzen bzw. unterkreuzen, so dass eine einheitliche Membran gebildet ist. Die luminale Funktionsschicht und die abluminale Stützschicht unterscheiden sich vorzugsweise durch ihre Porosität und die damit verbundene Funktion. Während die abluminale Stützschicht im Wesentlichen die Membran insgesamt stabilisieren soll und die Fasern der luminalen Funktionsschicht an ihrer vorbestimmten Position hält, dient die Funktionsschicht dazu, ein Aneurysma effizient von einem Blutstrom abzuschirmen. Gleichzeitig kann sich die Funktionsschicht jedoch für einen Blutstrom in ein abzweigendes Blutgefäß öffnen, um so die Blutversorgung von nachgelagerten Gewebearealen sicherzustellen.

Die Membran umfasst vorzugsweise zwar eine Funktionsschicht und eine Stützschicht. Dies schließt jedoch nicht aus, dass die Membran auch weitere Schichten, beispielweise zwei oder mehr Funktionsschichten und/oder zwei oder mehr Stützschichten und/oder weitere, andere Schichten, aufweist. Ferner kann die Membran eine Beschichtung, beispielsweise mit anti-thrombogenen Eigenschaften, aufweisen. Diese Beschichtung ist so vorgesehen, dass jeweils die einzelnen Fasern der Membran mit der Beschichtung umhüllt sind.

Um die Durchströmung der Funktionsschicht an Stellen sicherzustellen, an welchen ein Blutgefäß vom Hauptgefäß, in welches das Implantat eingesetzt ist, abzweigt, ist bei einer bevorzugten Variante der Erfindung vorgesehen, dass die Fasern der Membran, insbesondere zumindest die Fasern der Funktionsschicht, an Kreuzungspunkten lose aufeinander angeordnet sind, so dass sich kreuzende Fasern an den Kreuzungspunkten zueinander beweglich sind. Mit anderen Worten können die sich kreuzenden Fasern der inneren Membran aufeinander gleiten, wodurch sich die Poren, die durch die Fasern begrenzt sind, infolge des zuvor erläuterten Druckgradienten öffnen können. So kann ein Durchströmungsbereich geschaffen werden, durch welchen Blut in ein abzweigendes Blutgefäß geleitet werden kann.

Alternativ oder zusätzlich können die Fasern der Membran, insbesondere zumindest die Fasern der Funktionsschicht, elastisch und/oder plastisch verformbar sein, um infolge eines Druckgradienten auszuweichen und vergrößerte Poren zu formen, so dass lokal ein für die Blutversorgung nachgelagerter Gewebeareale ausreichender Blutfluss in ein abzweigendes Blutgefäß, insbesondere eine Arterie oder eine abzweigende Arteriole, erzielt wird.

Für die erfindungsgemäß vorteilhafte Funktion, dass sich die luminale Funktionsschicht abschnittsweise bzw. lokal für eine Blutdurchfuhr bzw. Flüssigkeitsdurchfuhr öffnen kann, ist es vorteilhaft, wenn die Funktionsschicht eine hohe Flexibilität aufweist. Insbesondere die Fasern der Funktionsschicht sollten möglichst flexibel sein, um eine Verformung zuzulassen, die zur Vergrößerung der Poren der Funktionsschicht führt. Die Vergrößerung der Poren erfolgt vorzugsweise zerstörungsfrei bzw. ohne Bildung von Faserrissen. Insofern ist es bevorzugt, wenn die Fasern der Funktionsschicht eine besonders geringe Faserdicke aufweisen. Die Faserdicke kann insbesondere weniger als 500 nm, insbesondere höchstens 400 nm, insbesondere höchstens 300 nm, insbesondere höchstens 200 nm, insbesondere höchstens 100 nm, aufweisen. Die abluminale Stützschicht, die eine stabilisierende Funktion aufweist, sollte hingegen stabilere Fasern umfassen. Dies kann dadurch erreicht werden, dass die Fasern der Stützschicht eine Faserdicke von mindestens 500 nm, insbesondere mindestens 750 nm, insbesondere mindestens 1000 nm, insbesondere mindestens 1250 nm, insbesondere mindestens 1500 nm, aufweisen.

Zur Funktion der Funktionsschicht, den Blutfluss in abzweigende Blutgefäße freizugeben, gleichzeitig jedoch ein Aneurysma effizient vom Blutfluss im Hauptgefäß abzuschirmen, trägt auch eine bevorzugte Gestaltung des medizinischen Implantats bei, bei welchem die Funktionsschicht eine Dicke von höchstens, insbesondere weniger als, 10 µm, insbesondere höchstens 8 µm, insbesondere höchstens 6 µm, insbesondere höchstens 4 µm, insbesondere höchstens 2 µm, aufweist. Dementsprechend trägt es zur Stabilisierungsfunktion der Stützschicht bei, wenn diese eine Dicke von mindestens 3 µm, insbesondere mindestens 5 µm, insbesondere mindestens 6 µm, insbesondere mindestens 7 µm, insbesondere mindestens 8 µm, aufweist.

Um ein Aneurysma effizient abschirmen zu können, ist eine besonders niedrige Porosität der Funktionsschicht vorteilhaft. Insofern ist bei einer bevorzugten Variante der Erfindung vorgesehen, dass die Funktionsschicht eine Porosität von weniger als 50%, insbesondere höchstens 40%, insbesondere höchstens 30%, aufweist. Die Stützschicht, die permanent blutdurchlässig sein soll, kann hingegen eine Porosität von mindestens 50%, insbesondere mindestens 60%, insbesondere mindestens 70%, insbesondere mindestens 80%, insbesondere mindestens 90%, aufweisen. Als Porosität wird im Rahmen der vorliegenden Anmeldung das Verhältnis zwischen der offenen Fläche eines Gewebes, also der Summe der Fläche aller Poren, zur Gesamtfläche dieses Gewebes verstanden.

Besonders bevorzugt ist eine Variante des medizinischen Implantats, bei welcher die Funktionsschicht auf einer Fläche von 100.000 µm² mindestens 10 Poren umfasst, die einen Inkreisdurchmesser von höchstens 10 µm, insbesondere höchstens 8 µm, insbesondere höchstens 6 µm, insbesondere höchstens 4 µm, insbesondere höchstens 2 µm, insbesondere höchstens 1 µm, aufweist. Alternativ oder zusätzlich kann die Stützschicht auf einer Fläche von 100.000 µm² mindestens 5 Poren, insbesondere mindestens 10 Poren, umfassen, die einen Inkreisdurchmesser von mindestens, insbesondere mehr als, 10 µm, insbesondere mindestens 15 µm, insbesondere mindestens 20 µm, insbesondere mindestens 25 µm, insbesondere mindestens 30 µm, insbesondere mindestens 40 µm, insbesondere mindestens 50 µm, insbesondere mindestens 60 µm, aufweisen.

Im Hinblick auf die unterschiedlichen Funktionen der Funktionsschicht und der Stützschicht, wobei die Funktionsschicht eine Flexibilität aufweisen soll, die bei einem entsprechenden Druckgradienten eine Durchströmung mit Blut ermöglicht, und die Stützschicht hingegen die Funktionsschicht stabilisieren soll, so dass sich diese nicht von der Trägerstruktur löst, ist es vorteilhaft, wenn die Fasern der Funktionsschicht eine kleinere Faserdicke als die Fasern der Stützschicht aufweisen.

Insofern ist es auch bevorzugt, wenn die Funktionsschicht bzw. deren Fasern eine höhere Dehnbarkeit als die Stützschicht bzw. deren Fasern aufweist.

Die Fasern der Funktionsschicht können aus einem Material gebildet sein, das eine niedrigere Shore-Härte als das Material der Fasern der Stützschicht aufweist. Insbesondere kann das Material der Fasern der Funktionsschicht eine Shore-Härte von höchstens 90A, insbesondere höchstens 80A, insbesondere höchstens 70A, insbesondere höchstens 60A, insbesondere höchstens 50A, und/oder das Material der Fasern der Stützschicht eine Shore-Härte von mindestens 90A, insbesondere mindestens 100A, insbesondere mindestens 60D, insbesondere mindestens 70D, insbesondere mindestens 80D, aufweisen.

Die Membran, insbesondere Funktionsschicht und die Stützschicht, kann ein thermoplastisches Polyurethan aufweisen. Das schließt nicht aus, dass die Funktionsschicht und/oder die Stützschicht jeweils weitere Kunststoffmaterialien umfassen. In einer bevorzugten Variante ist jedoch vorgesehen, dass die Funktionsschicht und die Stützschicht aus einem thermoplastischen Polyurethan bestehen. Es ist auch möglich, dass die Funktionsschicht oder die Stützschicht, insbesondere die Funktionsschicht und die Stützschicht bzw. die Membran insgesamt, aus einem absorbierbaren oder resorbierbaren Material gebildet ist. Insofern kann vorgesehen sein, dass sich die Funktionsschicht und/oder die Stützschicht über einen bestimmten Zeitraum durch den Kontakt mit Blut auflösen und so nach diesem Zeitraum nur noch die Trägerstruktur im Blutgefäß verbleibt. Vorzugsweise ist das absorbierbare oder resorbierbare Material so gewählt bzw. angepasst, dass es sich nach oder in einem Zeitraum auflöst, innerhalb dessen das durch die Funktionsschicht abgeschirmte Aneurysma verödet. Mit anderen Worten soll sich die Funktionsschicht und/oder die Stützschicht nicht auflösen, bevor das Aneurysma nicht verödet ist.

Die Fasern der Funktionsschicht können auch als konzentrische Fasern ausgebildet sein. Solche konzentrischen Fasern umfassen einen Faserkern und einen Fasermantel. Der Faserkern ist vorzugsweise durch ein weicheres Material als der Fasermantel gebildet, wobei der Fasermantel eine kleinere Dicke als der Faserkern umfasst. Der Fasermantel kann ein relative härteres Material aufweisen. Auf diese Weise kann über den Faserkern eine hohe Flexibilität der einzelnen Fasern erreicht werden, so dass die Fasern sich zur Freigabe von Poren infolge der Einwirkung eines Druckgradienten auf die Membran gut verformen lassen. Der Faserkern kann insofern ein Material mit einer Shore-Härte von höchstens 90A, insbesondere höchstens 80A, insbesondere höchstens 70A, insbesondere höchstens 60A, insbesondere höchstens 50A, und/oder das Material des Fasermantels eine Shore-Härte von mehr 90A, insbesondere mindestens 100A, insbesondere mindestens 60D, insbesondere mindestens 70D, insbesondere mindestens 80D, aufweisen. Das relativ härtere Material des Fasermantels dient hingegen dazu, ein gutes Gleiten der Fasern aufeinander zu ermöglichen, so dass auch über die Faserverschiebung eine gute Porenvergrößerung erreicht wird.

Die Membran kann sich bei einer bevorzugten Variante der Erfindung vollständig um den Umfang der Trägerstruktur erstrecken. Zwar ist es auch denkbar, dass sich die Membran lediglich teilweise um den Umfang der Trägerstruktur erstreckt, beispielsweise um bei Bifurkationsaneurysmen die Blutzufuhr von zuführenden Blutgefäß zu den beiden abzweigenden Gefäßen zu ermöglichen Die bevorzugte Variante, bei welcher sich die Membran vollständig um den Umfang der Trägerstruktur erstreckt, hat jedoch besondere Vorteile. Einerseits ist eine Herstellung des medizinischen Implantats in einer solchen Variante besonders einfach in Serienfertigung möglich. Andererseits bedingt die in Umfangsrichtung geschlossene Membran bereits eine Stabilisierung in sich, so dass sichergestellt ist, dass die Membran an der Trägerstruktur haftet und sich nicht von der Trägerstruktur löst. Insbesondere die Stützstruktur kann so ihre Stabilisierungsfunktion besonders effizient wahrnehmen.

Im Hinblick auf die Trägerstruktur sind unterschiedliche Varianten denkbar. Einerseits kann die Trägerstruktur monolithisch ausgebildet sein, wobei die Gitterelemente der Gitterstruktur Stege bilden, die als Zellen ausgebildete Gitteröffnungen der Gitterstruktur begrenzen. Mit anderen Worten kann die Trägerstruktur eine Gitterstruktur aufweisen, die aus einem rohrförmigen Ausgangsmaterial geschnitten ist. Dies kann beispielsweise durch Laserschneiden erfolgen. Durch das Schneiden des rohrförmigen Ausgangsmaterials entstehen Stege, die Zellen begrenzen. Andererseits kann die Trägerstruktur auch miteinander verflochtene Drähte aufweisen, wobei die Drähte die Gitterelemente der Gitterstruktur bilden und als Maschen ausgebildete Gitteröffnungen der Gitterstruktur begrenzen. Bei dieser Variante weist die Trägerstruktur also ein Geflecht aus miteinander verflochtenen Drähten auf, das die Gitterstruktur bildet. Die Drähte über- und unterkreuzen sich dabei, wobei zwischen den sich über- und unterkreuzenden Drähten Maschen ausgebildet werden. Es ist auch möglich, dass die Trägerstruktur aus Drahtelementen besteht oder Drahtelemente umfasst, die sich nicht überkreuzen (Wire Forming). Vielmehr können die Drahtelemente in einer gemeinsamen Umfangsebene angeordnet und miteinander verbunden sein, z.B. durch Schweißpunkte. Es ist auch möglich, die monolithische Gestaltung der Trägerstruktur durch eine Kombination aus Lithografie und einem Sputterverfahren, beispielsweise physikalische Gasphasenabscheidung (physical vapor deposition - PVD), insbesondere durch Magnetronsputtern, herzustellen.

Vorteilhaft für die Verwendung des erfindungsgemäßen medizinischen Implantats ist es, wenn dieses eine gute Biegeflexibilität aufweist und sich gut von einem möglichst kleinen komprimierten Zustand auf einen möglichst großen expandierten Zustand aufweiten lässt. Diese Aufweitung kann vorzugsweise selbsttätig erfolgen, indem entsprechende superelastische Materialien, beispielsweise Formgedächtnislegierungen, verwendet werden. Insoweit kann die Trägerstruktur insbesondere selbstexpandierbar sein. Das Implantat kann auch Formgedächtniskunststoffe aufweisen bzw. daraus bestehen.

Obwohl in einer bevorzugten Ausführungsform der Erfindung vorgesehen ist, dass die Gitterelemente der Trägerstruktur eine selbstexpandierbare Formgedächtnislegierung wie Nitinol umfassen oder daraus bestehen, ist es auch denkbar, die Trägerstruktur aus ballonexpandierbaren Materialien, wie Edelstahl oder CoCr-Legierungen, herzustellen. Letzteres ist insbesondere dann zweckmäßig, wenn die Trägerstruktur eine besonders kurze Länge aufweist und/oder eine besonders hohe Radialkraft aufweisen soll.

Eine gute Flexibilität des Implantats, sowohl im Hinblick auf die Biegeflexibilität, als auch im Hinblick auf die Expansionsfähigkeit, kann dadurch erreicht werden, dass das Verhältnis zwischen der Gesamtschichtdicke der Membran und der Höhe der Gitterelemente bzw. der Wandstärke der Trägerstruktur, entsprechend eingestellt wird. In einer besonders bevorzugten Variante weist die Membran eine Dicke auf, die höchstens 40%, insbesondere höchstens 30%, insbesondere höchstens 20%, insbesondere höchstens 10%, der Höhe der Gitterelemente, insbesondere der Stege oder der Drähte, beträgt.

Die Höhe der Gitterelemente entspricht bei einer Trägerstruktur, die monolithisch ausgebildet ist, der Höhe der Stege bzw. der Wandstärke der Trägerstruktur. Bei einer Trägerstruktur, die durch miteinander verflochtene Drähte gebildet ist, entspricht die Höhe der Gitterelemente der Drahtstärke. Die Gesamtwandstärke der Trägerstruktur weicht davon ab, da sich die Drähte stellenweise überkreuzen, so dass die Wandstärke der Trägerstruktur doppelt so hoch wie die Höhe der Gitterelemente, also der Drahtdurchmesser, ist. Jedenfalls ist vorgesehen, dass die Gesamtschichtdicke der Membran begrenzt ist, so dass sichergestellt ist, dass die Membran eine hohe Flexibilität aufweist, um einer Krümmung oder Expansion der Trägerstruktur gut folgen zu können.

Förderlich für die Gesamtflexibilität des Implantats ist es ferner, wenn, wie es bevorzugt vorgesehen ist, die Gitterelemente, insbesondere die Stege oder die Drähte, eine Höhe zwischen 40 µm und 160 µm, insbesondere zwischen 40 µm und 150 µm, insbesondere zwischen 40 µm und 130 µm, insbesondere zwischen 40 µm und 110 µm, insbesondere zwischen 40 µm und 100 µm, insbesondere zwischen 50 µm und 90 µm, insbesondere zwischen 50 µm und 80 µm, aufweisen.

Allgemein kann für bevorzugte Varianten des erfindungsgemäßen Implantats festgelegt sein, dass ein Verhältnis zwischen der Dicke der Membran und der Höhe der Gitterelemente, insbesondere der Stege oder der Drähte, höchstens 1/3, insbesondere höchstens 1/4, insbesondere höchstens 1/5, insbesondere höchstens 1/8, insbesondere höchstens 1/10, insbesondere höchstens 1/12, insbesondere höchstens 1/15, insbesondere höchstens 1/20, beträgt. Mit anderen Worten sind die Gitterelemente vorzugsweise doppelt bis zehnfach, insbesondere 3-mal bis 8-mal, insbesondere 4-mal bis 6-mal, so hoch, wie die Membran insgesamt dick ist.

Vorteilhaft ist es ferner, wenn das Implantat unter Röntgenkontrolle gut sichtbar ist. Das erleichtert es einem Operateur, die Position des Implantats im Blutgefäß zu ermitteln und zu prüfen, ob die Membran ihre Funktion (Abschirmung eines Aneurysmas aber gleichzeitig gute Perfusion von abzweigenden Blutgefäßen oder Arteriolen) wahrnimmt. Insofern kann vorgesehen sein, dass das Implantat zumindest teilweise oder lokal mit röntgensichtbaren Materialien versehen ist. Solche Materialien können Gold, Platin oder Tantal sowie Legierungen daraus sein.

Beispielsweise können an den Längsenden der Trägerstruktur Röntgenmarker, z.B. in Form von Ösen, Coils oder Hülsen, angeordnet sein. Insbesondere drei Röntgenmarker pro Längsende sind vorteilhaft, um das Implantat erkennbar zu machen. Es ist auch möglich, dass zusätzlich oder alternativ solche Röntgenmarker in einem mittleren Bereich der Trägerstruktur angeordnet sind. In die Trägerstruktur können auch zusätzliche Fäden eingewebt sein, die eine erhöhte Röntgensichtbarkeit aufweisen. Insbesondere kann ein solcher Faden entlang einer Reihe von miteinander fluchtenden Gitterelementen um diese gewickelt sein. Ein solcher Faden kann aus einem sogenannten DFT-Draht (drawn filled tube) gebildet sein.

Ferner ist es denkbar, dass zumindest einzelne Gitterelemente der Trägerstruktur ein röntgensichtbares Kernmaterial umfassen, das von einem Formgedächtnismaterial ummantelt ist (DFT-Draht). Ebenso können wenigstens einzelne, vorzugsweise alle, Fasern der Membran mit einem röntgensichtbaren Kernmaterial und einer Ummantelung aus einem anderen Material, z.B. einem Polyurethan, versehen sein. Zur Verbesserung der Röntgensichtbarkeit können röntgensichtbare Materialien auch zwischen der Funktionsschicht und der Stützschicht angeordnet sein. So kann beispielsweise wenigstens ein röntgensichtbares Vlies oder wenigstens ein röntgensichtbares Band zwischen der Funktionsschicht und der Stützschicht angeordnet sein. Schließlich ist es auch möglich, das röntgensichtbare Material, insbesondere Tantal, Niob, Platin oder Gold, mit Hilfe der Sputtertechnologie (insbesondere durch Magnetronsputtern) auf die Trägerstruktur aufzubringen bzw. in diese zu integrieren. Die Fasern können auch aus einem mit einem röntgendichten Material vermischten Kunststoff gebildet sein. So kann beispielsweise ein Kunststoff mit mindestens 20% Bariumsulfat gemischt sein, so dass die Membran zumindest bei Röntgen-Standbildern sichtbar ist.

In einer weiteren Ausführungsform der Erfindung ist vorgesehen, dass das Implantat mit einer antithrombogenen Beschichtung versehen ist, so dass jede Faser der Membran mit dieser Beschichtung umhüllt ist. Eine solche Beschichtung hat den Vorteil, dass die Poren der Membran, welche durch die Bewegung der Fasern der Membran entstehen, offen bleiben und nicht durch die Anlagerung von Blutplättchen verschlossen werden.

Vorzugsweise umfasst die Beschichtung eine Schichtdicke von höchstens 10 nm. Die Beschichtung kann Fibrin und/oder Heparin umfassen. Insbesondere kann die Beschichtung kovalent an Fibrin gebundenes Heparin aufweisen. Eine solche Beschichtung ist in der auf die Anmelderin zurückgehenden DE 10 2018 110 591 A1 beschrieben, auf die bezüglich der Zusammensetzung der Beschichtung Bezug genommen wird.

Im Allgemeinen kann die hier beschriebene Membran nicht nur (als Teil des erfindungsgemäßen Implantats) für die Behandlung von Aneurysmen eingesetzt werden, sondern auch für andere zerebral-vaskuläre Krankheiten Anwendung finden. Ein Beispiel dafür ist die Behandlung von arteriell-venösen Malformationen (sog. AVMs) und von arteriell-venösen Fisteln. Dabei kann Porosität und Flexibilität der Membran so eingestellt wird, dass der arteriell-venöse Kurzschluss die Venen zwar noch mit Blut versorgt, der Einstrom und damit der Druckaufbau in den Venen jedoch nicht mehr zu deren Ruptur führt.

Die Membran kann insgesamt durch ein Elektrospinning-Verfahren hergestellt sein. Beispielsweise können die Funktionsschicht und die Stützschicht unabhängig voneinander durch Elektrospinning herstellt und dann auf einer Trägerstruktur zusammengefügt werden. Durch die Vlies-artige Struktur der elektrogesponnenen Funktionsschicht und der elektrogesponnenen Stützschicht verbinden sich die beiden Schichten zu einer einheitlichen Membran, da Fasern der Stützschicht direkt durch Elektrospinning auf die Fasern der Funktionsschicht aufgebracht und damit stoffschlüssig verbunden werden. Die Prozessparameter und/oder Materialien für die Herstellung der Funktionsschicht und der Stützschicht unterscheiden sich, um die unterschiedlichen Funktionen der Funktionsschicht und der Stützschicht abzubilden. Es ist allerdings auch möglich, dass die Funktionsschicht und die Stützschicht in einem gemeinsamen Herstellungsschritt produziert werden. Hierzu kann ein Elektrospinning-Verfahren zum Einsatz kommen, bei dem unterschiedliche Materialien gleichzeitig abgeschieden werden, so dass unmittelbar die Membran entsteht, die einerseits die Funktion der Funktionsschicht und andererseits die Funktion der Stützschicht aufweist. Es ist allgemein angestrebt, dass die Dicke der Membran insgesamt, wie auch die Dicken der Funktionsschicht und der Stützschicht über die Länge der Membran im Wesentlichen konstant ist. Allerdings kann es auch sinnvoll sein, die vorgenannten Dicken über der Länge der Membran zu variieren.

Bei einer bevorzugten Weiterbildung der Erfindung ist vorgesehen, dass die Funktionsschicht im Bereich von Gitteröffnungen eine Perforation aufweist. Die Perforation kann insbesondere Löcher, gerade Schlitze, gekrümmte Schlitze und/oder T-förmige Schlitze aufweisen. Durch die Perforation werden in der Funktionsschicht Öffnungen erzeugt, die das lokale Öffnen der Funktionsschicht im Falle einer Druckdifferenz zwischen dem Flüssigkeitsdruck im inneren Durchgangskanal der Trägerstruktur und dem Flüssigkeitsdruck außerhalb der Stützschicht erleichtern. Die darüber angeordnete Stützschicht begrenzt den Öffnungsvorgang, so dass sich die Öffnung bzw. Perforation nicht zu weit aufdehnt. So kann sichergestellt werden, dass auch bei geringen Druckgradienten eine Öffnung erfolgt. Dabei ist die Perforation vorzugsweise so ausgebildet, dass eine lokale Öffnung der Funktionsschicht in Bereichen erfolgt, die einem Druckgradienten ausgesetzt sind, der zwischen einem Hauptblutgefäß und einem abzweigenden Blutgefäß oder einem Perforatorgefäß üblicherweise vorliegt. Insbesondere ist die Perforation so eingestellt, dass eine Öffnung der Funktionsschicht in Bereichen unterbleibt, wenn ein geringerer Druckgradient vorliegt, beispielsweise bei einem Druckgradienten, der sich üblicherweise zwischen einem Hauptblutgefäß und einem Aneurysma ausbildet. Allerdings soll eine lokale Öffnung der Funktionsschicht erfolgen, um Blut in ein Perforatorgefäß strömen zu lassen.

Die Perforation kann durch Laserbearbeitung der Membran, insbesondere der Funktionsschicht, und/oder durch Lösungsmittel-Sprühen hergestellt werden.

Bei der Laserbearbeitung kann die Funktionsschicht insbesondere mittels eines UV-Lasers oder eines Femto- oder Picosekunden-Infrarotlasers mit einem Perforationsmuster versehen werden. Als Perforationsmuster kommen insbesondere Löcher, gerade Schlitze bzw. Schnitte, kiemenartige, gekrümmte Schlitze und/oder T-förmige Schlitze bzw. -schnitte in Betracht. Eine musterartige Verteilung der Perforation über einen größeren Bereich der Membran ist generell bevorzugt. Beim Einsetzen des Implantats ist so sichergestellt, dass vor ein etwaig überdecktes Seitengefäß eine Perforation zu liegen kommt und somit ein Flüssigkeitsdurchtritt durch die Membran in das Seitengefäß ermöglicht ist.

Beim Lösungsmittel-Sprühen wird ein Nebel aus feinen Tropfen eines Lösungsmittels bzw. Lösungsmittel-Polymer-Gemischs in einer definierten Größe erzeugt. Beim Auftreffen des Nebels auf die Funktionsschicht werden die Fasern der Funktionsschicht aufgelöst und so Löcher in der Funktionsschicht gebildet.

Ferner ist es denkbar, durch eine Maskierung bei der Herstellung der Membran, insbesondere der Funktionsschicht, eine Perforation zu erzeugen. So kann die Funktionsschicht so maskiert werden, dass bei der Bildung der Funktionsschicht durch einen Sprühprozess ein Perforationsmuster erstellt bzw. freigehalten wird. Diese Herstellungsvariante eignet sich besonders für die Bildung von Löchern als Perforationsmuster.

Das erfindungsgemäße medizinische Implantat ist vorzugsweise durch ein Verfahren herstellbar, das die folgenden Schritte aufweist:
a. Bereitstellen der Trägerstruktur;
b. Aufbringen der Funktionsschicht auf die Trägerstruktur;
c. Perforieren der Funktionsschicht durch einen Laserschneidprozess oder durch Lösungsmittelsprühen; und
d. Aufbringen der Stützschicht auf die Funktionsschicht.

Alternativ zur Bildung der Perforation mittels eines Laserschneidprozesses oder mittels Lösungsmittelsprühen kann die Funktionsschicht auch dadurch mit der Perforation versehen werden, dass beim Aufbringen der Funktionsschicht einzelne Areale freigelassen werden. Dies kann beispielsweise durch eine Maske erfolgen, die vor dem Aufsprühen der Funktionsschicht auf die Trägerstruktur aufgelegt und nach dem Aufsprühen der Funktionsschicht wieder entfernt wird.

Das beschriebene Verfahren ermöglicht eine einfache und effiziente Herstellung eines Implantats mit einer intelligenten Membran, die sich infolge eines entsprechend hohen Druckgradienten bereichsweise öffnen kann, um einen Flüssigkeitsdurchtritt zu ermöglichen.

Im Übrigen kann vorgesehen sein, dass zwischen der Trägerstruktur und der Funktionsschicht eine weitere Stützschicht angeordnet ist. Die weitere Stützschicht kann Fasern mit einer Faserdicke aufweisen, die größer als die Faserdicke der Fasern der Funktionsschicht ist. Die Dichte der Fasern der Stützschicht kann überdies kleiner als die Dichte der Fasern der Funktionsschicht sein.

Die Erfindung wird im Folgenden anhand von Ausführungsbeispielen unter Bezugnahme auf die beigefügten schematischen Zeichnungen näher erläutert. Darin zeigen:
- Figur 1: einen Ausschnitt aus einem Blutgefäßsystem, in dem ein erfindungsgemäßes medizinisches Implantat eingesetzt ist;
- Figur 2: einen Detailausschnitt des Implantats gemäß Figur 1 bei Überdeckung eines Aneurysmas;
- Figur 3: einen Detailausschnitt des Implantats gemäß Figur 1 bei Überdeckung eines abzweigenden Blutgefäßes; und
- Fig. 4 bis 7: jeweils eine Seitenansicht erfindungsgemäßer medizinischer Implantats nach bevorzugten Ausführungsbeispielen, bei welchen die Funktionsschicht jeweils eine andere Perforation aufweist.

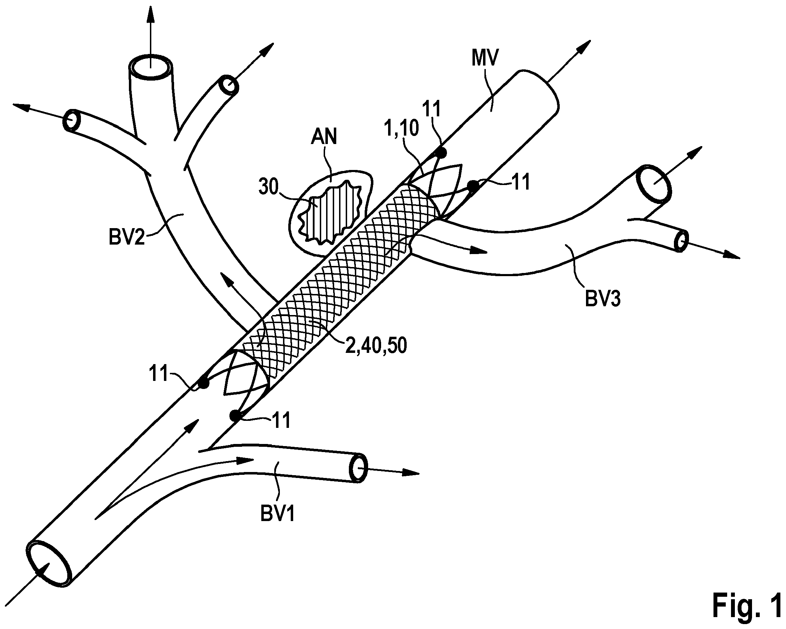

Figur 1 zeigt einen Ausschnitt aus einem Blutgefäßsystem mit einem Hauptgefäß MV und drei vom Hauptgefäß MV abzweigenden Seitengefäßen BV1, BV2, BV3. Das Hauptgefäß MV weist außerdem ein Aneurysma AN auf, das zwischen dem zweiten Seitengefäß BV2 und dem dritten Seitengefäß BV3 angeordnet ist. Insbesondere ist das Aneurysma nahe des dritten Seitengefäßes BV3 positioniert.

Zur Behandlung des Aneurysmas AN wird das erfindungsgemäße medizinische Implantat eingesetzt. Das medizinische Implantat umfasst eine Trägerstruktur 1, die durch eine Gitterstruktur 10 aus Gitterelementen gebildet ist. Die Gitterelemente können Stege 12 sein, die einstückig miteinander verbunden sind und so die Gitterstruktur 10 bilden. Dabei begrenzen die Stege 12 Zellen 13 der Gitterstruktur 10. Alternativ kann die Gitterstruktur 10 auch durch miteinander verflochtene Drähte gebildet sein. Um die Gitterstruktur 10 bzw. die Trägerstruktur 1 unter Röntgenkontrolle beim Einsatz des Implantats in das Blutgefäßsystem bzw. in das Hauptgefäß MV sichtbar zu machen, sind an den Längsenden der Gitterstruktur 10 jeweils Röntgenmarker 11 vorgesehen. Vorzugsweise sind an jedem Längsende der Gitterstruktur 10 mehrere Röntgenmarker 11 angeordnet, die in Umfangsrichtung der Gitterstruktur 10 in regelmäßigen Abständen positioniert sind.

Das Implantat weist ferner eine Membran 2 auf, die eine luminalen Funktionsschicht 4 und eine abluminale Stützschicht 3 umfasst. Die Funktionsschicht 4 und die Stützschicht 3 überlappen sich vorzugsweise vollständig, weisen also in Längsrichtung der Gitterstruktur 10 dieselbe Länge auf. Bevorzugt ist es jedoch, wenn die Stützschicht 3 die Funktionsschicht 4 zumindest an den Längsenden überragt, vorzugsweise um wenige Millimeter. Wie in Figur 1 erkennbar ist, kann die Gitterstruktur 10 länger als die Membran 2 sein.

Das Implantat ist derart im Hauptgefäß MV angeordnet, dass das Implantat, insbesondere die Membran 2, den Hals des Aneurysmas AN vollständig überdeckt. Im Aneurysma AN kann außerdem ein Embolisationsmittel 30 angeordnet werden. Konkret kann das medizinische Implantat in einem bzw. als Set gemeinsam mit einem Embolisationsmittel 30 angeboten werden. Das Embolisationsmittel 30 kann beispielsweise ein Gel sein. Alternativ kann das Embolisationsmittel 30 auch durch Coils, also sich chaotisch windende Mikrodrähte, gebildet sein. Das Embolisationsmittel 30 kann in das Aneurysma AN eingebracht werden, nachdem das Implantat in das Hauptgefäß MV eingesetzt wurde. Beispielsweise können Coils über einen Mikrokatheter durch die Membran 2 hindurch in das Aneurysma zugeführt werden. Die Membran 2 ist bzw. deren Fasern sind dabei so flexibel, dass der Mikrokatheter die Poren der Membran 2 aufweiten und sich so einen Weg in das Aneurysma AN bahnen kann.

Wie in der Figur 1 ebenfalls erkennbar ist, überspannt die Membran 2 nicht nur das Aneurysma AN, sondern auch das zweite Seitengefäß BV2 und das dritte Seitengefäß BV3. Hier kommt die besondere Funktion der Membran 2 zum Tragen. Die Membran 2 umfasst die abluminale Stützschicht 3, die eine größere Porosität aufweist als die luminale Funktionsschicht 4. Die Stützschicht 3 ist dabei derart porös, dass sie permanent blutdurchlässig ist. Die Funktionsschicht 4 ist hingegen ist im Wesentlichen gering blutdurchlässig, insbesondere semipermeabel, und vor allem weniger blutdurchlässig als die Stützschicht 3. Gleichzeitig ist die Funktionsschicht 4 jedoch derart flexibel, dass sie bei entsprechender Kraftbeaufschlagung blutdurchlässig oder stärker blutdurchlässig wird. Eine solche erforderliche Kraft kann durch den Druckgradienten entstehen, der sich zwischen dem Blutdruck im Hauptgefäß MV und dem abnehmenden Blutdruck in einem der Seitengefäße BV1, BV2, BV3 einstellt.

Da die Funktionsschicht die Blutströmung in ein Seitengefäß BV1, BV2, BV3 zunächst reduziert, entsteht ein Druckgefälle bzw. ein stärkeres Druckgefälle zwischen dem Blutdruck im Hauptgefäß MV und dem entsprechenden Seitengefäß BV1, BV2, BV3. Dieses Druckgefälle bzw. dieser Druckgradient bewirkt eine Kraft, die ausreichend hoch ist, um die Poren der der Funktionsschicht 4 zu aufzuweiten. Dies erfolgt dadurch, dass die Fäden der Funktionsschicht 4 elastisch und/oder plastisch verformt werden und/oder aufeinander gleiten, so dass ausschließlich im Bereich des abzweigenden Blutgefäßes, also lokal in Bereich der Öffnung in das entsprechende Seitengefäß BV1, BV2, BV3, die Funktionsschicht 4 blutdurchlässig bzw. stärker blutdurchlässig wird. Die Membran 2 ist insoweit "intelligent", da sie nur an denjenigen Stellen den Blutdurchfluss gewährt, an welchen der Druckgradient zwischen dem Blutdruck im Hauptgefäß MV und einem Druck außerhalb der äußeren Membran 50 ausreichend hoch ist. Diese Schwelle wird regelmäßig lokal an Stellen der Membran 2 überschritten, die vom Hauptgefäß MV abzweigende Seitengefäße BV1, BV2, BV3 überdecken. An der Stelle der Membran 2, die das sich vom Hauptgefäß MV ausgehend öffnende Aneurysma AN überspannt, wird die Druckschwelle nicht überschritten, d.h. der Druckgradient zwischen dem Blutdruck im Hauptgefäß MV und einem Druck innerhalb des Aneurysmas AN ist nicht ausreichend groß, um die Poren der Funktionsschicht 4 aufzuweiten. So bleibt das Aneurysma AN vom Blutstrom abgeschirmt, so dass das im Aneurysma AN verbleibende Blut in kurzer Zeit gerinnt und das Aneurysma AN so verödet.

Wenn, wie es bei dem Ausführungsbeispiel gemäß Figur 1 vorgesehen ist, zusätzlich ein Embolisationsmittel 30 im Aneurysma angeordnet ist, so bewirkt die Stützschicht 3, die im Wesentlichen eine stabilisierende Funktion aufweist, auch, dass das Embolisationsmittel 30 im Aneurysma AN gehalten wird, sich also nicht zurück in das Hauptgefäß MV bewegt. Dies sorgt zusätzlich dafür, dass das Aneurysma AN zeitnah verödet.

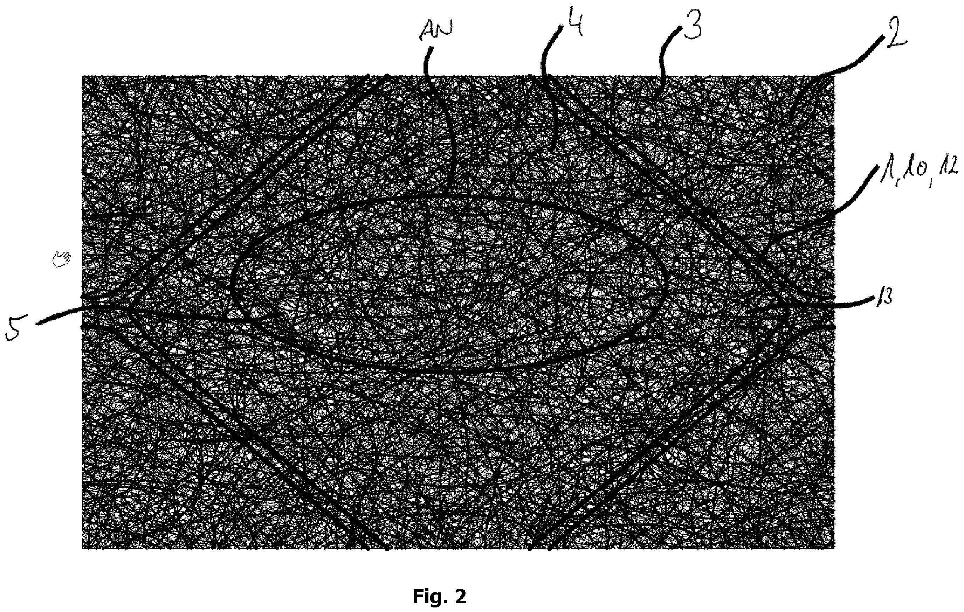

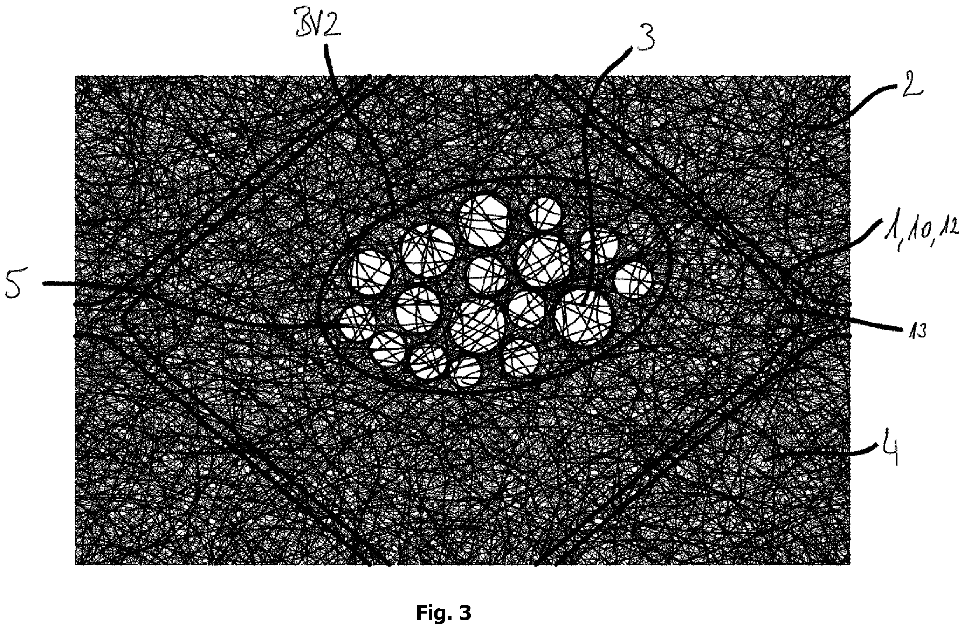

Die Figuren 2 und 3 zeigen jeweils einen Ausschnitt des Implantats mit einer Trägerstruktur 1 und einer Membran 2. Die Trägerstruktur 1 ist durch eine Gitterstruktur 10 gebildet, wobei in den Figuren 2 und 3 jeweils einige Stege 12 der Gitterstruktur gezeigt sind. Die Stege 12 bilden Zellen 13 der Gitterstruktur 10. Bei dem dargestellten Ausführungsbeispiel sind die Stege 12 monolithisch miteinander verbunden. Die Gitterstruktur 10 ist folglich einstückig ausgebildet. Es ist jedoch auch möglich, dass die Gitterstruktur 10 durch Drähte gebildet ist, die miteinander verflochten oder verwebt sind.

Die Zelle 13 wird durch die Membran 2 überspannt. Die Membran 2 umfasst mindestens zwei Schichten, die jeweils durch elektrogesponnene Fäden gebildet sind. Die Schichten unterscheiden sich durch die Dicke und die Dichte der Fäden.

Konkret weist die Membran 2 eine Stützschicht 3 auf, die eine relativ geringere Dichte an Fäden mit relativ höherer Fadendicke aufweist. Die Stützschicht 3 unterscheidet sich somit von einer Funktionsschicht 4, deren Fäden eine kleinere Fadendicke aufweisen. Ferner ist die Dichte der Fäden der Funktionsschicht 4 höher als die Dichte der Fäden der Stützschicht 3. Mit anderen Worten weisen die Stützschicht 3 und die Funktionsschicht 4 jeweils von den Fäden begrenzte Poren 5 auf, die in der Stützschicht 3 größer als in der Funktionsschicht 4 sind. Dies gilt jedenfalls für den Ruhezustand des Implantats, d.h. ohne äußere Krafteinwirkung.

Die Funktionsschicht 4 hat die Aufgabe, einen Blutfluss durch die Membran 2 zu behindern oder zumindest zu verlangsamen. Die Funktionsschicht 4 wirkt insofern als Flow Diverter, lenkt den Blutfluss also entlang ihrer Oberfläche ab. Wegen der geringen Fadendicke ist die Funktionsschicht relativ flexibel. Die Stützschicht 3 stabilisiert die Funktionsschicht 4 und verhindert so, dass sich die Funktionsschicht 4 in radialer Richtung auswölbt, bzw. bewirkt, dass die Funktionsschicht 4 eng an der Trägerstruktur 1 anliegt.

Figur 2 zeigt das Prinzip der Ablenkung des Blutflusses. Die Membran 2, die sich über die Zelle 13 erstreckt, überspannt ein Aneurysma AN. Zwischen dem Aneurysma AN und dem Hauptgefäß MV, in welches das Implantat eingesetzt ist, besteht kaum ein relevanter Druckgradient, so dass die Funktionsschicht 4 im Wesentlichen ihren Ruhezustand beibehält. Folglich weist die Funktionsschicht 4 eine kleine Porengröße auf, so dass der Blutfluss hauptsächlich entlang der Funktionsschicht 4 geleitet wird und nicht wesentlich in das Aneurysma AN eindringt. Das Aneurysma AN ist somit vom Blutfluss im Hauptgefäß MV weitgehend abgekoppelt und kann durch Gerinnung des im Aneurysma AN verbleibenden Blutes veröden. Dennoch kann durch die Poren der Membran ein geringer Blutfluss in das Aneurysma strömen, damit der Gerinnungsprozess und die Bildung eines soliden Thrombus im Aneurysma nicht unterbrochen werden.

Figur 3 zeigt die Funktion der Funktionsschicht 4, wenn diese ein abzweigendes Blutgefäß, bspw. das zweite Seitengefäß BV2, überspannt. Durch die Druckdifferenz, die sich zwischen dem Hauptgefäß MV und dem zweiten Seitengefäß BV2 einstellt, werden die Fäden der Funktionsschicht 4 ausgelenkt bzw. lokal verformt. Dadurch vergrößern sich im Bereich der Mündung des zweiten Seitengefäßes BV2 die Poren der Funktionsschicht 4. Die Fäden der Stützschicht 3 sind hingegen stabiler und behalten ihre Position weitgehend bei. Die Poren der Stützschicht 3 sind jedoch ohne hin groß genug, um einen Blutfluss durch die Stützschicht 3 zu erlauben. So reicht es aus, dass sich die Poren der Funktionsschicht 4 im Bereich der Mündung des zweiten Seitengefäßes BV2 erweitern, um einen ausreichenden Blutfluss vom Hauptgefäß MV in das zweite Seitengefäß BV2 zu ermöglichen.

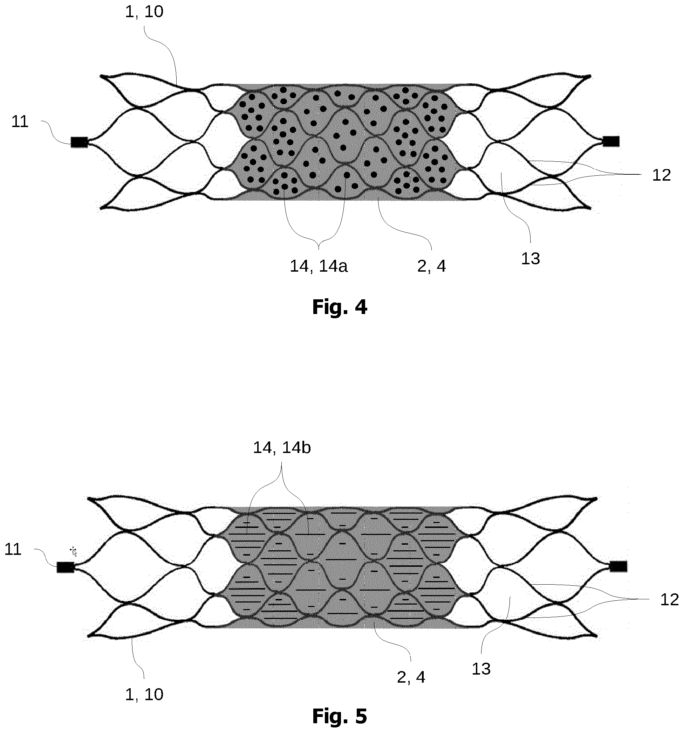

In den Figuren 4 bis 7 sind verschiedene Ausführungsbeispiele medizinischer Implantate gezeigt, deren Funktionsschicht 4 mit einer Perforation 14 ausgestattet ist. Aus Gründen der Übersichtlichkeit ist in den Figuren 4 bis 7 die Stützschicht 3 nicht dargestellt.

Konkret zeigen die Figuren 4 bis 7 jeweils einen Stent mit einer Trägerstruktur 1, die als Gitterstruktur 10 ausgebildet ist. Die Gitterstruktur 10 umfasst mehrere einstückig gekoppelte Stege 12, die Zellen 13 begrenzen. An den Längsenden der Gitterstruktur 10 sind Röntgenmarker 11 angeordnet. In einem mittleren Bereich der Gitterstruktur 10 ist eine Membran 2 mit einer Funktionsschicht 4 vorgesehen. Die Membran 2 erstreckt sich über den gesamten Umfang der Gitterstruktur 10 und überdeckt die Zellen 13 vollständig. Die Membran 2 ist mit den Stegen 12 der Gitterstruktur 10 verbunden.

Die in den Figuren 4 bis 7 dargestellte Funktionsschicht 4 weist eine Perforation 14 auf. Die Perforation 14 ist vorzugsweise musterartig verteilt über die Funktionsschicht 4 angeordnet. Insbesondere liegt die Perforation 14 im Bereich von Gitteröffnungen bzw. Zellen 13 der Trägerstruktur 1 vor. Im Hinblick auf die musterartige Anordnung der Perforation 14 bestehen Gemeinsamkeiten bei den Ausführungsbeispielen gemäß Figuren 4 bis 7. So ist bei den dargestellten Ausführungsbeispielen vorgesehen, dass die Dichte der Perforationen 14 in Zellen 13, die nahe am Längsende der Funktionsschicht 4 angeordnet sind, höher als in Zellen 13 eines mittleren Bereichs der Funktionsschicht 4 ist. Bei der Positionierung des Implantats im Bereich eines Aneurysmas AN soll sichergestellt sein, dass der Blutfluss in das Aneurysma AN zu einem bestimmten Grad unterbrochen wird. Eine lokale Öffnung der Funktionsschicht 4 im Bereich des Aneurysmas AN ist also unerwünscht. Üblicherweise wird das Implantat so positioniert, dass der mittlere Bereich des Implantats, insbesondere der Funktionsschicht 4, im Bereich des Aneurysmas AN zu liegen kommt. Indem in diesem Bereich dennoch, wenngleich weniger dicht gepackte, Perforation 14 vorliegt, können z.B. dem Aneurysma AN gegenüber abzweigende Seitengefäße BV1, BV2, BV3 dennoch gut mit Nährstoffen versorgt werden, weil die Perforation 14 eine Öffnung der Funktionsschicht 4 erlaubt. In den Randbereichen der Funktionsschicht 4 ist die Wahrscheinlichkeit höher, dass hiermit ein Seitengefäß BV1, BV2, BV3 abgedeckt wird. Die dort vorgesehene Perforation 14 hat eine größere Durchlässigkeit.

Die Ausführungsbeispiele 4 bis 7 unterscheiden sich durch die Art der Perforation 14 der Funktionsschicht 4. So zeigt Figur 4 ein Ausführungsbeispiel, bei dem die Funktionsschicht 4 eine durch Löcher 14a gebildete Perforation 14 aufweist. Die Löcher 14a befinden sich im Wesentlichen in Bereichen der Funktionsschicht 4, die die Gitteröffnungen bzw. Zellen 13 bedecken. Die Anzahl der Löcher 14a ist in den Zellen 13, die an den Längsenden der Funktionsschicht 4 angeordnet sind größer, als in einem mittleren Bereich der Funktionsschicht 4.

Bei dem Ausführungsbeispiel gemäß Figur 5 ist die Perforation 14 durch gerade Schlitze 14b gebildet, die sich parallel zur Längsachse der Gitterstruktur 10 erstrecken. Eine andere Ausrichtung der geraden Schlitze 14b ist möglich. Insbesondere können die geraden Schlitze 14b bezogen auf eine in die Wandungsebene des Implantats projizierte Längsachse des Implantats unter einem Winkel zwischen 0° und 180° angeordnet sein.

Die Länge der Schlitze 14b ist bei dem Ausführungsbeispiel gemäß Figur 5 an den verfügbaren Raum zwischen in Längsrichtung der Gitterstruktur 10 benachbarten Stegen 12 angepasst, so dass die Schlitze 14b unterschiedliche Längen aufweisen. Der Abstand der Schlitze 14b in Umfangsrichtung der Gitterstruktur 10 variiert ebenfalls, wobei in Randbereichen der Funktionsschicht 4 ein geringerer Abstand als in einem mittleren Bereich der Funktionsschicht 4 vorgesehen ist.

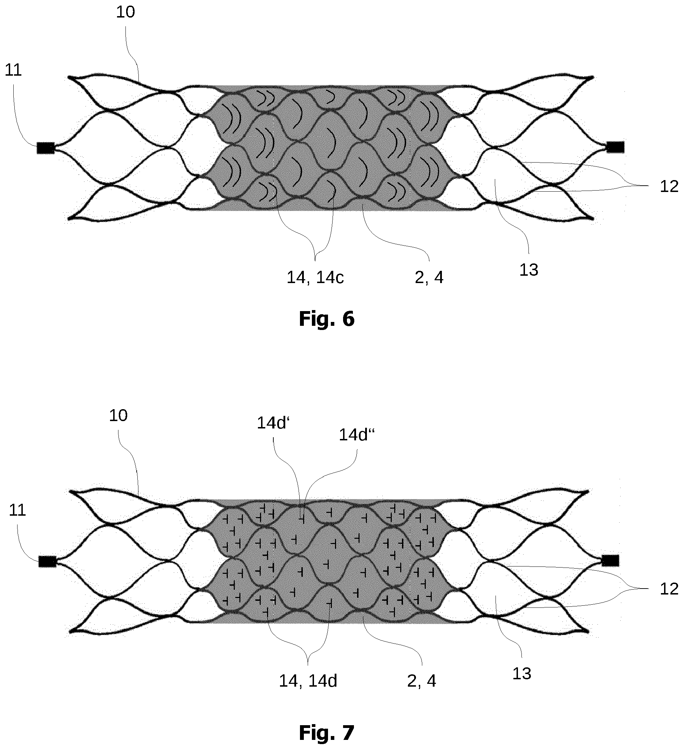

Das Ausführungsbeispiel gemäß Figur 6 zeigt ein Implantat mit einer Funktionsschicht 4, die eine Perforation 14 aus gekrümmten Schlitzen 14c aufweist. Die gekrümmten Schlitze 14c erstrecken sich im Wesentlichen in Umfangsrichtung der Gitterstruktur 10. In den Randbereichen der Funktionsschicht 4 sind in jeder Zelle 13 zwei gekrümmte Schlitze 14c angeordnet, wobei in einem mittleren Bereich der Funktionsschicht 4 jeder Zelle 13 ein gekrümmter Schlitz 14c zugeordnet ist. Eine andere Anzahl und Verteilung der Perforation 14 ist möglich.

Die gekrümmten Schlitze 14c sind vorzugsweise in dieselbe Richtung und insbesondere in Strömungsrichtung des Blutes ausgerichtet. Mit anderen Worten wird das Implantat gemäß Figur 6 vorzugsweise so in einem Blutgefäß platziert, dass das Blut in von den Längsenden der gekrümmten Schlitze 14c zum Scheitelpunkt deren Krümmung strömt. In der Darstellung gemäß Figur 6 würde das Blut also vom linken Ende der Gitterstruktur 10 zum rechten Ende der Gitterstruktur 10 fließen. Die gekrümmten Schlitze 14c bilden insofern kiemenartige Öffnungen in der Funktionsschicht 4.

Generell gilt für alle Ausführungsbeispiele, bei welchen eine Perforation 14 vorgesehen ist, dass die Stützschicht 3 eine Rückhaltefunktion für das Öffnen der Perforation 14 aufweist. Die Perforation öffnet insbesondere bei der kiemenartigen Ausführung der Öffnungen durch Auslenken eines Teils der Funktionsschicht 4. Diese Auslenkung wird durch die Stützschicht 3 begrenzt, die insoweit eine Rückhaltefunktion für die ventilartige Öffnung der Perforation 14 hat. Die Rückhaltefunktion der Stützschicht 3 und die Perforation 14 der Funktionsschicht 4 sind daher so aufeinander abgestimmt, dass sich die Perforation 14 nur dann öffnet, wenn ein vorbestimmter Druckgradient zwischen der Innenseite der Membran 2 und der Außenseite der Membran 2 vorliegt.

Figur 7 zeigt ein Ausführungsbeispiel, bei welchem die Perforation 14 der Funktionsschicht 4 durch T-förmige Schlitze 14d gebildet ist. Auch bei diesem Ausführungsbeispiel sind in den Randbereichen der Funktionsschicht 4 mehr T-förmige Schlitze 14d pro Zelle 13 vorgesehen als in einem mittleren Bereich der Funktionsschicht 4. Die T-förmigen Schlitze 14d sind vorzugsweise in dieselbe Richtung ausgerichtet. Jeder T-förmige Schlitz 14d umfasst insbesondere einen Stammschlitz 14d' und einen Balkenschlitz 14d", wobei sich der Stammschlitz 14d' parallel zur Längsachse der Gitterstruktur 10 und der Balkenschlitz 14d" senkrecht dazu erstrecken. Der Balkenschlitz 14d" schließt an einem distalen Längsende des Stammschlitzes 14d' an. Das Implantat wird vorzugsweise so im Blutgefäß platziert, dass das Blut vom proximalen Ende des Stammschlitzes 14d' zum Balkenschlitz 14d" strömt.

### Bezugszeichenliste

- 1: Trägerstruktur
- 2: Membran
- 3: Stützschicht
- 4: Funktionsschicht
- 5: Pore
- 10: Gitterstruktur
- 11: Röntgenmarker
- 12: Steg
- 13: Zelle
- 14: Perforation
- 14a: Loch
- 14b: gerader Schlitz
- 14c: gekrümmter Schlitz
- 14d: t-förmiger Schlitz
- 14d': Stammschlitz
- 14d": Balkenschlitz
- 30: Embolisationsmittel
- AN: Aneurysma
- BV1: erstes Seitengefäß
- BV2: zweites Seitengefäß
- BV3: drittes Seitengefäß
- MV: Hauptgefäß

## Patentansprüche

1. Medizinisches Implantat zur Behandlung von Aneurysmen mit einer Trägerstruktur (1), die eine komprimierbare und expandierbare Gitterstruktur (10) aus Gitterelementen aufweist, welche Gitteröffnungen begrenzen, wobei die Gitterstruktur (10) zumindest abschnittsweise mit einer, insbesondere elektrogesponnenen, Membran (2) aus Fasern abgedeckt ist, die mindestens eine luminale Funktionsschicht (4) und mindestens eine abluminale Stützschicht (3) umfasst, die jeweils Poren aufweisen, wobei die Porosität der Funktionsschicht (4) kleiner als die Porosität der Stützschicht (3) ist,
wobei die Membran (2) so konfiguriert ist, dass sich zumindest die Poren der Funktionsschicht (4) infolge eines Druckgradienten, der sich zwischen einem Flüssigkeitsdruck in einem inneren Durchgangskanal der Trägerstruktur (1) und einem Flüssigkeitsdruck außerhalb der Stützschicht (3) einstellt, zur Erhöhung des Flüssigkeitsdurchflusses durch die Membran (2) öffnen.

2. Medizinisches Implantat nach Anspruch 1,
**dadurch gekennzeichnet, dass**
die Fasern der Membran (2), insbesondere der luminalen Funktionsschicht (4), an Kreuzungspunkten lose aufeinander angeordnet sind, so dass sich kreuzende Fasern an den Kreuzungspunkten zueinander beweglich sind, und/oder dass zumindest die Fasern der Funktionsschicht (4) der Membran (2) elastisch und/oder plastisch verformbar sind.

3. Medizinisches Implantat nach einem der vorhergehenden Ansprüche,
**dadurch gekennzeichnet, dass**
die Fasern der Funktionsschicht (4) der Membran (2) eine Faserdicke von weniger als 500 nm, insbesondere höchstens 400 nm, insbesondere höchstens 300 nm, insbesondere höchstens 200 nm, insbesondere höchstens 100 nm, aufweist und/oder dass die Fasern der Stützschicht (3) der Membran (2) eine Faserdicke von mindestens 500 nm, insbesondere mindestens 750 nm, insbesondere mindestens 1000 nm, insbesondere mindestens 1250 nm, insbesondere mindestens 1500 nm, aufweisen.

4. Medizinisches Implantat nach einem der vorhergehenden Ansprüche,
**dadurch gekennzeichnet, dass**
die Funktionsschicht (4) der Membran (2) eine Dicke von höchstens, insbesondere weniger als, 10 µm, insbesondere höchstens 8 µm, insbesondere höchstens 6 µm, insbesondere höchstens 4 µm, insbesondere höchstens 2 µm, aufweist und/oder dass die Stützschicht (3) der Membran (2) eine Dicke von mindestens 3 µm, insbesondere mindestens 5 µm, insbesondere mindestens 6 µm, insbesondere mindestens 7 µm, insbesondere mindestens 8 µm, aufweist.

5. Medizinisches Implantat nach einem der vorhergehenden Ansprüche,
**dadurch gekennzeichnet, dass**
die Funktionsschicht (4) der Membran (2) eine Porosität von weniger als 50%, insbesondere höchstens 40%, insbesondere höchstens 30%, aufweist und/oder dass die Stützschicht (3) der Membran (2) eine Porosität von mindestens 50%, insbesondere mindestens 60%, insbesondere mindestens 70%, insbesondere mindestens 80%, insbesondere mindestens 90%, aufweist.

6. Medizinisches Implantat nach einem der vorhergehenden Ansprüche,
**dadurch gekennzeichnet, dass**
die Funktionsschicht (4) der Membran (2) auf einer Fläche von 100.000 µm² mindestens 10 Poren umfasst, die einen Inkreisdurchmesser von höchstens 10 µm, insbesondere höchstens 8 µm, insbesondere höchstens 6 µm, insbesondere höchstens 4 µm, insbesondere höchstens 2 µm, insbesondere höchstens 1 µm, aufweisen
und/oder dass die Stützschicht (3) der Membran (2) auf einer Fläche von 100.000 µm² mindestens 5 Poren umfasst, die einen Inkreisdurchmesser von mindestens, insbesondere mehr als, 10 µm, insbesondere mindestens 15 µm, insbesondere mindestens 20 µm, insbesondere mindestens 25 µm, insbesondere mindestens 30 µm, insbesondere mindestens 40 µm, insbesondere mindestens 50 µm, insbesondere mindestens 60 µm, aufweisen.

7. Medizinisches Implantat nach einem der vorhergehenden Ansprüche,
**dadurch gekennzeichnet, dass**
die Fasern der Funktionsschicht (4) eine kleinere Faserdicke als die Fasern der Stützschicht (3) aufweisen und/oder dass die Fasern der Funktionsschicht (4) eine höhere Dehnbarkeit als die Stützschicht (3) aufweisen.

8. Medizinisches Implantat nach einem der vorhergehenden Ansprüche,
**dadurch gekennzeichnet, dass**
die Fasern der Funktionsschicht (4) aus einem Material gebildet sind, das eine niedrigere Shore-Härte als das Material der Fasern der Stützschicht (3) aufweist, insbesondere wobei das Material der Fasern der Funktionsschicht (4) eine Shore-Härte von höchstens 90A, insbesondere höchstens 80A, insbesondere höchstens 70A, insbesondere höchstens 60A, insbesondere höchstens 50A, und/oder das Material der Fasern der Stützschicht (3) eine Shore-Härte von mindestens 90A, insbesondere mindestens 100A, insbesondere mindestens 60D, insbesondere mindestens 70D, insbesondere mindestens 80D, aufweist.

9. Medizinisches Implantat nach einem der vorhergehenden Ansprüche,
**dadurch gekennzeichnet, dass**
die Membran (2), insbesondere jeweils die Funktionsschicht (4) und die Stützschicht (3), ein thermoplastisches Polyurethan aufweisen oder daraus bestehen, wobei sich die Membran (2) vorzugsweise vollständig um den Umfang der Trägerstruktur (1) erstreckt.

10. Medizinisches Implantat nach einem der vorhergehenden Ansprüche,
**dadurch gekennzeichnet, dass**
die Trägerstruktur (1) monolithisch ausgebildet ist, wobei die Gitterelemente der Gitterstruktur (10) Stege (12) bilden, die als Zellen (13) ausgebildete Gitteröffnungen der Gitterstruktur (10) begrenzen.

11. Medizinisches Implantat nach einem der vorhergehenden Ansprüche,
**dadurch gekennzeichnet, dass**
die Trägerstruktur (1) miteinander verflochtene Drähte aufweist, wobei die Drähte die Gitterelemente der Gitterstruktur (10) bilden und als Maschen ausgebildete Gitteröffnungen der Gitterstruktur (10) begrenzen.

12. Medizinisches Implantat nach Anspruch 11,
**dadurch gekennzeichnet, dass**
die Membran (2) eine Gesamtschichtdicke aufweist, die höchstens 40%, insbesondere höchstens 30%, insbesondere höchstens 20%, insbesondere höchstens 10%, der Höhe der Gitterelemente, insbesondere der Stege (12) oder der Drähte, beträgt und/oder die Gitterelemente, insbesondere die Stege (12) oder die Drähte, vorzugsweise eine Höhe zwischen 40 µm und 160 µm, insbesondere zwischen 40 µm und 150 µm, insbesondere zwischen 40 µm und 130 µm, insbesondere zwischen 40 µm und 110 µm, insbesondere zwischen 40 µm und 100 µm, insbesondere zwischen 50 µm und 90 µm, insbesondere zwischen 50 µm und 80 µm, aufweisen und wobei ein Verhältnis zwischen der Dicke der Membran (2) und der Höhe der Gitterelemente, insbesondere der Stege (12) oder der Drähte, vorzugsweise höchstens 1/3, insbesondere höchstens 1/4, insbesondere höchstens 1/5, insbesondere höchstens 1/8, insbesondere höchstens 1/10, insbesondere höchstens 1/12, insbesondere höchstens 1/15, insbesondere höchstens 1/20, beträgt.

13. Medizinisches Implantat nach einem der vorhergehenden Ansprüche,
**dadurch gekennzeichnet, dass**
die Funktionsschicht (4) im Bereich von Gitteröffnungen eine Perforation aufweist.

14. Medizinisches Implantat nach Anspruch 13,
**dadurch gekennzeichnet, dass**
die Perforation durch Löcher (14a), gerade Schlitze (14b), gekrümmte Schlitze (14c) und/oder Kreuzschlitze gebildet ist.

15. Verfahren zur Herstellung eines medizinischen Implantat nach einem der vorhergehenden Ansprüche, mit den folgenden Schritten:
a. Bereitstellen der Trägerstruktur (1);
b. Aufbringen der Funktionsschicht (4) auf die Trägerstruktur;
c. Perforieren der Funktionsschicht (4) durch einen Laserschneidprozess; und
d. Aufbringen der Stützschicht (3) auf die Funktionsschicht (4).

## Claims

1. A medical implant for the treatment of aneurysms, with a carrier structure (1) which has a compressible and expandable mesh structure (10) produced from mesh elements which delimit mesh openings, wherein the mesh structure (10) is covered, at least in sections, with a membrane (2) which is produced from fibres and which in particular is electrospun and which comprises at least one luminal functional layer (4) and at least one abluminal support layer (3) which respectively have pores, wherein the porosity of the functional layer (4) is smaller than the porosity of the support layer (3), wherein the membrane (2) is configured in a manner such that, as a consequence of a pressure gradient which occurs between a liquid pressure in an inner through channel of the carrier structure (1) and a liquid pressure outside the support layer (3), at least the pores of the functional layer (4) open in order to increase the flow of liquid through the membrane (2).

2. The medical implant as claimed in claim 1,
**characterized in that**
the fibres of the membrane (2), in particular of the luminal functional layer (4), are disposed loosely on top of one another at points of intersection, so that intersecting fibres are movable with respect to each other at the points of intersection, and/or **in that** at least the fibres of the functional layer (4) of the membrane (2) are elastically and/or plastically deformable.

3. The medical implant as claimed in one of the preceding claims,
**characterized in that**
the fibres of the functional layer (4) of the membrane (2) have a fibre thickness of less than 500 nm, in particular at most 400 nm, in particular at most 300 nm, in particular at most 200 nm, in particular at most 100 nm, and/or **in that** the fibres of the support layer (3) of the membrane (2) have a fibre thickness of at least 500 nm, in particular at least 750 nm, in particular at least 1000 nm, in particular at least 1250 nm, in particular at least 1500 nm.

4. The medical implant as claimed in one of the preceding claims,
**characterized in that**
the functional layer (4) of the membrane (2) has a thickness of at most, in particular less than, 10 µm, in particular at most 8 µm, in particular at most 6 µm, in particular at most 4 µm, in particular at most 2 µm, and/or **in that** the support layer (3) of the membrane (2) has a thickness of at least 3 µm, in particular at least 5 µm, in particular at least 6 µm, in particular at least 7 µm, in particular at least 8 µm.

5. The medical implant as claimed in one of the preceding claims,
**characterized in that**
the functional layer (4) of the membrane (2) has a porosity of less than 50 %, in particular at most 40 %, in particular at most 30 %, and/or **in that** the support layer (3) of the membrane (2) has a porosity of at least 50 %, in particular at least 60 %, in particular at least 70 %, in particular at least 80 %, in particular at least 90%.

6. The medical implant as claimed in one of the preceding claims,
**characterized in that**
the functional layer (4) of the membrane (2) comprises at least 10 pores which have an inscribed circle diameter of at most 10 µm, in particular at most 8 µm, in particular at most 6 µm, in particular at most 4 µm, in particular at most 2 µm, in particular at most 1 µm, over a surface area of 100000 µm², and/or **in that** the support layer (3) of the membrane (2) comprises at least 5 pores which have an inscribed circle diameter of at least, in particular more than, 10 µm, in particular at least 15 µm, in particular at least 20 µm, in particular at least 25 µm, in particular at least 30 µm, in particular at least 40 µm, in particular at least 50 µm, in particular at least 60 µm, over a surface area of 100000 µm².

7. The medical implant as claimed in one of the preceding claims,
**characterized in that**
the thickness of the fibres of the functional layer (4) is smaller than the fibres of the support layer (3), and/or **in that** the fibres of the functional layer (4) have a higher ductility than the support layer (3).

8. The medical implant as claimed in one of the preceding claims,
**characterized in that**
the fibres of the functional layer (4) are formed from a material which has a lower Shore hardness than the material of the fibres of the support layer (3), in particular wherein the material of the fibres of the functional layer (4) has a Shore hardness of at most 90A, in particular at most 80A, in particular at most 70A, in particular at most 60A, in particular at most 50A, and/or the material of the fibres of the support layer (3) has a Shore hardness of at least 90A, in particular at least 100A, in particular at least 60D, in particular at least 70D, in particular at least 80D.

9. The medical implant as claimed in one of the preceding claims,
**characterized in that**
the membrane (2), in particular the functional layer (4) and the support layer (3) respectively, comprise or consist of a thermoplastic polyurethane, wherein the membrane (2) preferably extends around the entire circumference of the carrier structure (1).

10. The medical implant as claimed in one of the preceding claims,
**characterized in that**
the carrier structure (1) is monolithic in configuration, wherein the mesh elements of the mesh structure (10) form webs (12) which delimit mesh openings of the mesh structure (10) which are formed as cells (13).

11. The medical implant as claimed in one of the preceding claims,
**characterized in that**
the carrier structure (1) has interwoven wires, wherein the wires form the mesh elements of the mesh structure (10) and delimit mesh openings of the mesh structure (10) which are formed as loops.

12. The medical implant as claimed in claim 11, **characterized in that**
the membrane (2) has a total layer thickness which is at most 40 %, in particular at most 30 %, in particular at most 20 %, in particular at most 10 % of the height of the mesh elements, in particular the webs (12) or the wires, and/or the height of the mesh elements, in particular the webs (12) or the wires, is preferably a height of between 40 µm and 160 µm, in particular between 40 µm and 150 µm, in particular between 40 µm and 130 µm, in particular between 40 µm and 110 µm, in particular between 40 µm and 100 µm, in particular between 50 µm and 90 µm, in particular between 50 µm and 80 µm, and wherein a ratio between the thickness of the membrane (2) and the height of the mesh elements, in particular the webs (12) or the wires, is preferably at most 1/3, in particular at most 1/4, in particular at most 1/5, in particular at most 1/8, in particular at most 1/10, in particular at most 1/12, in particular at most 1/15, in particular at most 1/20.

13. The medical implant as claimed in one of the preceding claims,
**characterized in that**
the functional layer (4) has a perforation in the region of mesh openings.

14. The medical implant as claimed in claim 13,
**characterized in that**
the perforation is formed by holes (14a), straight slits (14b), curved slits (14c) and/or cross-slits.

15. A method for the production of a medical implant as claimed in one of the preceding claims, with the following steps:
a. providing the carrier structure (1);
b. applying the functional layer (4) to the carrier structure;
c. perforating the functional layer (4) by a laser cutting process; and
d. applying the support layer (3) to the functional layer (4).

## Revendications

1. Implant médical pour le traitement d'anévrismes, comportant une structure porteuse (1) qui présente une structure grillagée comprimable et expansible (10) composée d'éléments de grille qui délimitent des ouvertures de grille, la structure grillagée (10) étant recouverte du moins par sections d'une membrane (2), en particulier électrofilée, en fibres qui comprend au moins une couche fonctionnelle luminale (4) et au moins une couche support abluminale (3) qui présentent respectivement des pores, la porosité de la couche fonctionnelle (4) étant inférieure à la porosité de la couche support (3),
la membrane (2) étant configurée de manière à ce qu'au moins les pores de la couche fonctionnelle (4) s'ouvrent à la suite d'un gradient de pression qui s'établit entre une pression de liquide dans un canal de passage intérieur de la structure porteuse (1) et une pression de liquide à l'extérieur de la couche support (3) pour augmenter le débit de liquide à travers la membrane (2) .

2. Implant médical selon la revendication 1,
**caractérisé en ce que** les fibres de la membrane (2), en particulier de la couche fonctionnelle luminale (4), sont disposées en vrac les unes sur les autres à des points de croisement de manière à ce que des fibres se croisant aux points de croisement soient mobiles les unes par rapport aux autres, et/ou à ce qu'au moins les fibres de la couche fonctionnelle (4) de la membrane (2) soient déformables élastiquement et/ou plastiquement.

3. Implant médical selon une des revendications précédentes,
**caractérisé en ce que**
les fibres de la couche fonctionnelle (4) de la membrane (2) présentent une épaisseur de fibres de moins de 500 nm, en particulier d'au plus 400 nm, en particulier d'au plus 300 nm, en particulier d'au plus 200 nm, en particulier d'au plus 100 nm, et/ou que les fibres de la couche support (3) de la membrane (2) présentent une épaisseur de fibres d'au moins 500 nm, en particulier d'au moins 750 nm, en particulier d'au moins 1000 nm, en particulier d'au moins 1250 nm, en particulier d'au moins 1500 nm.

4. Implant médical selon une des revendications précédentes,
**caractérisé en ce que**
la couche fonctionnelle (4) de la membrane (2) présente une épaisseur d'au plus, en particulier moins de, 10 µm, en particulier d'au plus 8 µm, en particulier d'au plus 6 µm, en particulier d'au plus 4 µm, en particulier d'au plus 2 µm, et/ou que la couche support (3) de la membrane (2) présente une épaisseur d'au moins 3 µm, en particulier d'au moins 5 µm, en particulier d'au moins 6 µm, en particulier d'au moins 7 µm, en particulier d'au moins 8 µm.

5. Implant médical selon une des revendications précédentes,
**caractérisé en ce que**
la couche fonctionnelle (4) de la membrane (2) présente une porosité de moins de 50 %, en particulier d'au plus 40 %, en particulier d'au plus 30 %, et/ou que la couche support (3) de la membrane (2) présente une porosité d'au moins 50 %, en particulier d'au moins 60 %, en particulier d'au moins 70 %, en particulier d'au moins 80 %, en particulier d'au moins 90 %.

6. Implant médical selon une des revendications précédentes,
**caractérisé en ce que**
la couche fonctionnelle (4) de la membrane (2), sur une surface de 100 000 µm², comprend au moins 10 pores, qui présentent un diamètre intérieur d'au plus 10 µm, en particulier d'au plus 8 µm, en particulier d'au plus 6 µm, en particulier d'au plus 4 µm, en particulier d'au plus 2 µm, en particulier d'au plus 1 µm, et/ou que la couche support (3) de la membrane (2), sur une surface de 100 000 m², comprend au moins 5 pores, qui présentent un diamètre intérieur d'au moins, en particulier plus de, 10 µm, en particulier d'au moins 15 µm, en particulier d'au moins 20 µm, en particulier d'au moins 25 µm, en particulier d'au moins 30 µm, en particulier d'au moins 40 µm, en particulier d'au moins 50 µm, en particulier d'au moins 60 µm.

7. Implant médical selon une des revendications précédentes,
**caractérisé en ce que**
les fibres de la couche fonctionnelle (4) présentent une épaisseur de fibres inférieure à celle des fibres de la couche support (3) et/ou que les fibres de la couche fonctionnelle (4) présentent une extensibilité supérieure à celle de la couche support (3).

8. Implant médical selon une des revendications précédentes,
**caractérisé en ce que**
les fibres de la couche fonctionnelle (4) sont constituées d'un matériau qui présente une dureté Shore plus faible que le matériau des fibres de la couche support (3), le matériau des fibres de la couche fonctionnelle (4) présentant en particulier une dureté Shore d'au plus 90A, en particulier d'au plus 80A, en particulier d'au plus 70A, en particulier d'au plus 60A, en particulier d'au plus 50A, et/ou le matériau des fibres de la couche support (3) présentant une dureté Shore d'au moins 90A, en particulier d'au moins 100A, en particulier d'au moins 60D, en particulier d'au moins 70D, en particulier d'au moins 80D.

9. Implant médical selon une des revendications précédentes,
**caractérisé en ce que**
la membrane (2), en particulier respectivement la couche fonctionnelle (4) et la couche support (3), présentent un polyuréthane thermoplastique ou en sont composées, la membrane (2) s'étendant de préférence entièrement autour de la circonférence de la structure porteuse (1).

10. Implant médical selon une des revendications précédentes,
**caractérisé en ce que**
la structure porteuse (1) a une conformation monolithique, les éléments de grille de la structure grillagée (10) formant des traverses (12) qui délimitent les ouvertures de grille réalisées sous forme de cellules (13) de la structure grillagée (10).

11. Implant médical selon une des revendications précédentes,
**caractérisé en ce que**
la structure porteuse (1) présente des fils entrelacés, les fils formant les éléments de grille de la structure grillagée (10) et délimitant les ouvertures de grille réalisées sous forme de mailles de la structure grillagée (10).

12. Implant médical selon la revendication 11, **caractérisé en ce que**
la membrane (2) présente une épaisseur de couche totale qui représente au plus 40 %, en particulier au plus 30 %, en particulier au plus 20 %, en particulier au plus 10 %, de la hauteur des éléments de grille, en particulier des traverses (12) ou des fils et/ou que les éléments de grille, en particulier les traverses (12) ou les fils présentent une hauteur comprise de préférence entre 40 µm et 160 µm, en particulier entre 40 µm et 150 µm, en particulier entre 40 µm et 130 µm, en particulier entre 40 µm et 110 µm, en particulier entre 40 µm et 100 µm, en particulier entre 50 µm et 90 µm, en particulier entre 50 µm et 80 µm, et un rapport entre l'épaisseur de la membrane (2) et la hauteur des éléments de grille, en particulier des traverses (12) ou des fils, est de préférence d'au plus 1/3, en particulier d'au plus 1/4, en particulier d'au plus 1/5, en particulier d'au plus 1/8, en particulier d'au plus 1/10, en particulier d'au plus 1/12, en particulier d'au plus 1/15, en particulier d'au plus 1/20.

13. Implant médical selon une des revendications précédentes,
**caractérisé en ce que**
la couche fonctionnelle (4) présente une perforation au niveau des ouvertures de grille.

14. Implant médical selon la revendication 13,
**caractérisé en ce que**
la perforation est constituée par des trous (14a), des fentes droites (14b), des fentes courbées (14c) et/ou des fentes en croix.

15. Procédé de fabrication d'un implant médical selon une des revendications précédentes, comportant les étapes suivantes :
a. prévision de la structure porteuse (1) ;
b. application de la couche fonctionnelle (4) sur la structure porteuse ;
c. perforation de la couche fonctionnelle (4) par un processus de découpe au laser ; et
d. application de la couche support (3) sur la couche fonctionnelle (4).
